# EUROPEAN PATENT APPLICATION

(11) **EP 2 902 361 A1**
(43) Date of publication of application: **05.08.2015**
(21) Application number: 13842064.1
(22) Date of filing: 27.08.2013
(51) Int. Cl.: C01B 21/064, A61K 8/19, A61Q 1/06, A61Q 1/10, A61Q 1/12

(54) **HIGHLY WATER-REPELLENT AND HIGHLY OIL-ABSORBENT BORON NITRIDE POWDER, PRODUCTION METHOD THEREFOR, AND COSMETIC**

(30) Priority: 28.09.2012 JP 2012218413
(71) Applicant: Mizushima Ferroalloy Co. Ltd., Okayama 712-8513 (JP)
(72) Inventor: KOSHIDA, Takahisa, Kurashiki-shi Okayama 712-8513 (JP); KUMAGAI, Masato, Kurashiki-shi Okayama 712-8513 (JP); HIWASA, Shoichi, Kurashiki-shi Okayama 712-8513 (JP)
(74) Representative: Carpmaels & Ransford LLP
(86) International application number: PCT/JP2013/005048
(87) International publication number: WO 2014/049956

(57) **Abstract**

A boron nitride powder including flat-shaped primary particles of BN and an aggregate of the primary particles has a water permeation speed less than 1 mm²/s and oil absorption of 100 ml/100 g to 500 ml/100 g, which is a cosmetic boron nitride powder excellent in water repellency and oil absorbency. The use of such a boron nitride powder provides a cosmetic significantly improved not only in gloss finish and transparency (bare skin feeling) but also in sustainability.

## Description

### TECHNICAL FIELD

The present invention relates to a highly water repellent and highly oil absorbent boron nitride powder and a method for manufacturing the same, and is intended to significantly improve the makeup sustainability in its application to a cosmetic.

The present invention also relates to a cosmetic using the boron nitride powder.

The highly water repellent and highly oil absorbent boron nitride powder according to the present invention is particularly suitable for use in an oil-based cosmetic such as powder foundation among cosmetics. Such an oil-based cosmetic adheres well even when sweating and the like and lasts for a long time once applied, and so is advantageous in that the frequency of makeup reapplication can be reduced.

### BACKGROUND

A boron nitride powder (also referred to as a BN powder) has excellent lubricity as compared with other materials, and is gaining attention as a pigment for cosmetics (also referred to as cosmetic products). In particular, for its excellent lubricity, the boron nitride powder is increasingly used as a cosmetic extender recently.

A cosmetic extender is a base for dispersing a color pigment, and significantly influences feelings of use such as "spreadability" (the property of being smoothly applied to the skin surface) and "sustainability" (the property of sustaining the state of application to the skin).

Conventional cosmetic extenders are mostly natural ores and resins, which are not always satisfactory in terms of usability, stability, etc. For example, inorganic materials such as talc, mica, and sericite have catalytic activities, which can degrade perfumes or oils and cause smell change. Resin materials such as nylon powder and polyethylene powder are chemically stable, but have a problem of poor formability.

The BN powder is excellent in spreadability and sustainability, as compared with natural ores and resins.

This is because the BN powder not only has excellent lubricity, but also is flat-shaped and so has appropriate coverage and adhesion.

JP H5-186205 A (Patent Literature (PTL) 1) and JP H7-41311 A (PTL 2) propose manufacturing methods for such BN powders. These manufacturing methods are expected to supply chemically stable, flat-shaped boron nitride powders.

### CITATION LIST

### Patent Literatures

PTL 1: JP H5-186205 A
PTL 2: JP H7-41311 A

As mentioned above, a BN powder is increasingly used instead of conventional materials as it exhibits excellent effects of improving the properties of basic cosmetics.

Meanwhile, cosmetic users' desire to further enhance the evenness of application to the skin to make the skin look more beautiful is growing more and more. This spurs the development of various high-function new materials.

With such ongoing development of various high-function new materials, there is demand to develop a cosmetic extender that is excellent in durability of effect, in particular sustainability, in use as an extender.

The present invention advantageously meets this demand, and has an object of providing a boron nitride powder for cosmetics that achieves significantly improved sustainability as compared with conventional techniques, together with an advantageous method for manufacturing the same.

The present invention also has an object of providing a cosmetic that has significantly improved sustainability by use of the above-mentioned boron nitride powder, as compared with conventional techniques.

### SUMMARY

As a result of conducting intensive study to fulfill the objects stated above, the present inventors have discovered that the sustainability of powder foundation is closely related to the surface characteristics of a BN powder contained. The present inventors have thus found that the sustainability can be significantly improved by making the surface of the BN powder highly water repellent and highly oil absorbent.

In detail, the sustainability of a cosmetic product is closely related to the oil absorption of the constitutional powder of the cosmetic.

For example, when secretions such as sweat are produced from the skin, the state on the skin surface differs greatly depending on the oil absorption of the powder surface. If the oil absorption of the powder is low, the powder does not adhere well due to sweating, which leads to poor sustainability. If the oil absorption of the powder is high, the powder adheres without floating, which leads to improved sustainability. Therefore, to improve the sustainability, the oil absorption of the powder, i.e. the BN powder, needs to be improved.

A BN powder with higher water repellency tends to have higher oil absorption. Accordingly, reducing functional groups on the powder surface is effective.

The BN powder uses an oxide such as boron oxide or boric acid as a raw material, and so there is a possibility that boron remains in the end product. In the case where the BN powder in which boron remains is used in a cosmetic, boron may leach and damage the skin.

In an existing process, boron which is an impurity is reduced by washing. In the case where an organic dispersant is used to enhance the washing efficiency, however, a large amount of organic functional groups remain on the surface of the BN powder. This makes it impossible to attain high water repellency.

Heating treatment is effective in reducing the functional groups that remain on the surface as a result of washing. Heating the BN powder at high temperature in the atmosphere, however, causes BN oxidation and results in a decrease in water repellency.

In view of this, the present inventors have conducted various studies on a method for advantageously improving the water repellency by removing the functional groups on the surface without oxidizing the BN powder.

As a result, the present inventors have found that the desired object can be fulfilled by performing heating treatment in a non-oxidizing, reduced-pressure atmosphere.

The present invention is based on these findings.

In detail, the present invention basically has the following structure.
1. A highly water repellent and highly oil absorbent boron nitride powder including flat-shaped primary particles of BN and an aggregate of the primary particles, wherein a water permeation speed is less than 1 mm²/s, and oil absorption is 100 ml/100 g to 500 ml/100 g.
2. The highly water repellent and highly oil absorbent boron nitride powder according to the foregoing 1, wherein the primary particles of BN are flat-shaped with an average major diameter of 2 µm to 20 µm and a thickness of 0.05 µm to 0.5 µm.
3. The highly water repellent and highly oil absorbent boron nitride powder according to the foregoing 1 or 2, wherein an amount of soluble boron is less than or equal to 100 ppm.
4. The highly water repellent and highly oil absorbent boron nitride powder according to any of the foregoing 1 to 3, wherein a specific surface area is 1 m²/g to 10 m²/g, and an oxygen content is less than or equal to 1.5 mass%.
5. A method for manufacturing a highly water repellent and highly oil absorbent boron nitride powder, the method including: heating at least one of boric acid and a dehydration product thereof, at least one of urea and a compound thereof, and boron carbide in an inert atmosphere to obtain a boron nitride powder having a turbostratic structure; heat-treating the obtained boron nitride powder at a temperature of 1500 °C to 2300 °C in an inert atmosphere; grinding and then washing the heat-treated boron nitride powder to remove boric acid; and heat-treating the washed boron nitride powder at a temperature of 300 °C or higher in a non-oxidizing, reduced-pressure atmosphere with a furnace pressure less than or equal to 0.01 MPa.
6. A cosmetic including the boron nitride powder according to any of the foregoing 1 to 4.
7. The cosmetic according to the foregoing 6, wherein an amount of the boron nitride powder contained in the cosmetic is 0.1 mass% to 70 mass%.
8. The cosmetic according to the foregoing 6 or 7, wherein the cosmetic is powder foundation.

The boron nitride powder according to the present invention has excellent lubricity, and has the property of spreading like sliding with a light force. Hence, smooth extension can be achieved in inunction operation when using the cosmetic.

Moreover, the cosmetic according to the present invention has improved water repellency and oil absorbency, and so has improved resistance to sweat and the like secreted from the skin. This leads to significant improvements in sustainability, gloss finish, and transparency (bare skin feeling).

### BRIEF DESCRIPTION OF THE DRAWINGS

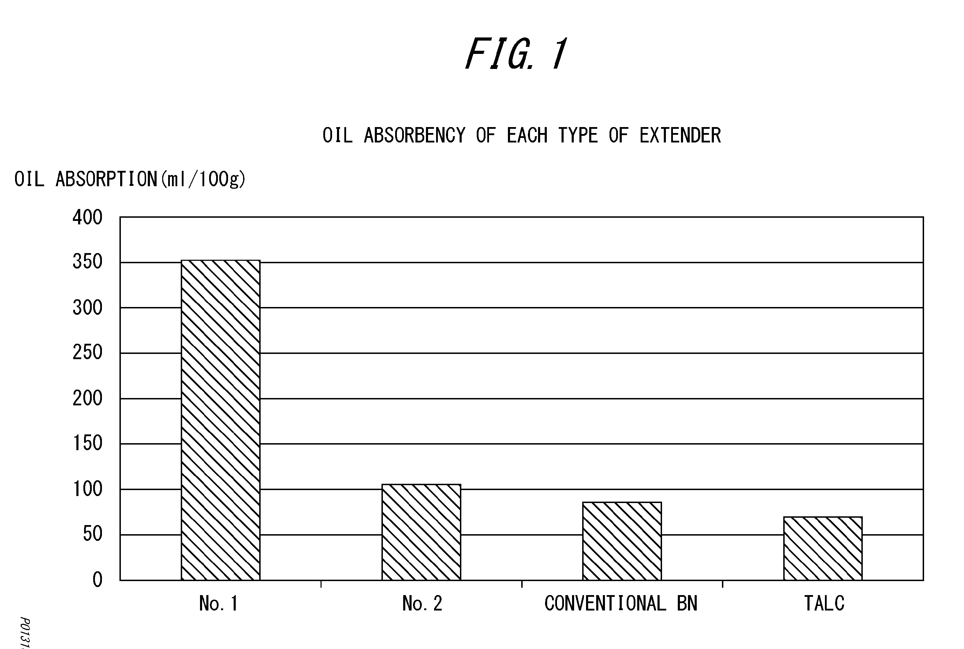

The present invention will be further described below with reference to the accompanying drawings, wherein
FIG. 1 is a diagram illustrating the oil absorbency of a BN powder according to the present invention in comparison with talc which is a conventional typical extender and a conventional BN powder.

### DETAILED DESCRIPTION

The following describes the present invention in detail.

A boron nitride powder according to the present invention is basically made up of flat-shaped primary particles, and is characterized by high water repellency and high oil absorption.

The water repellency is described first.

Various methods have been proposed for water repellency evaluation. A typical method is to evaluate the water repellency from the angle of contact between a powder and a liquid. With this evaluation method, however, quantitative evaluation is difficult, though qualitative evaluation of tendencies is possible.

In view of this, in the present invention, a water permeability test conforming to JIS A 6909 (Water Permeability Test B Method) that enables quantitative evaluation is employed, and the water permeation speed is specified to be less than 1 mm²/s in the water permeability test. If the permeation speed is not less than 1 mm²/s, functional groups are inevitably present on the surface of the BN powder, and satisfactorily high water repellency cannot be obtained. The permeation speed is more preferably less than or equal to 0.8 mm²/s. The lower limit of the permeation speed is not particularly limited, and may be 0.

A water repellent BN powder has a feature that there are few functional groups on its surface. A typical method of evaluating the functional groups on the surface is based on analysis on the amount of impurities. The impurities are mostly oxygen and carbon. Of these, oxygen is present on the surface of the BN powder as OH groups or carbonyl groups, and lowers the water repellency and oil absorbency of the powder.

Moreover, since the leaching of B significantly damages the skin, it is desirable to reduce the amount of soluble B to less than or equal to 100 ppm. The amount of soluble B correlates with the specific surface area of the powder, and tends to increase when the specific surface area exceeds 10 m²/g. It is therefore preferable to set the specific surface area to less than or equal to 10 m²/g. Besides, if the specific surface area is larger, the surface activity increases, and the interparticle bond strength increases to create strongly aggregated particles. This causes a disadvantage of increased rough feeling. In this respect, too, it is preferable to set the specific surface area to less than or equal to 10 m²/g. If the specific surface area is below 1 m²/g, the particle diameter is excessively large, and a problem of degradation in feeling of use such as moist feeling or gloss arises. Hence, the specific surface area of the BN powder aggregate is preferably in a range from 1 m²/g to 10 m²/g. The specific surface area of the BN powder aggregate is more preferably in a range from 2 m²/g to 5 m²/g.

If the oxygen content in the BN powder exceeds 1.5 mass%, boron oxide as an impurity increases. This causes a disadvantage such as damage to the skin in the case where such BN is used in a cosmetic. Accordingly, the oxygen content is preferably less than or equal to 1.5 mass%, and more preferably less than or equal to 1.0 mass%.

Equally from a safety point of view, the pH of the BN powder is preferably neutral in a range from about 5 to 9. The pH of the BN powder is measured according to the Japanese Standards of Quasi-drug Ingredients 2006 (Yakuji Nippo Limited).

The oil absorption is described next.

The oil absorption is a factor closely related to the finish and sustainability of the cosmetic, and higher oil absorption is more preferable.

The oil absorption of talc or a conventional BN powder is only about 80 ml/100 g. In the present invention, on the other hand, by performing heating treatment in a non-oxidizing, reduced-pressure atmosphere, the water repellency and thus the oil absorption of the BN powder can be increased to 100 ml/100 g or more. Here, since excessively high oil absorption causes a problem of large variations in viscosity, bulk density, and the like of the compound when manufacturing the cosmetic, the upper limit of the oil absorption is set to 500 ml/100 g. The oil absorption is preferably in a range from 150 ml/100 g to 400 ml/100 g.

FIG. 1 illustrates the oil absorbency of the BN powders (Nos. 1 and 2) according to the present invention in comparison with talc which is a conventional typical extender and a conventional BN powder. The BN powders (Nos. 1 and 2) according to the present invention correspond to Nos. 1 and 2 in Table 1 described later.

As illustrated in the drawing, the BN powders according to the present invention have significantly improved oil absorbency as compared with the conventional BN powder or talc.

The primary particles of BN according to the present invention are preferably flat-shaped with an average major diameter of 2 µm to 20 µm and a thickness of 0.05 µm to 0.5 µm.

Primary particles below 2 µm in average major diameter are difficult to be manufactured. Meanwhile, primary particles exceeding 20 µm exhibit orientation, which causes degradation in feeling of use such as moist feeling or gloss in the case of use in a cosmetic. If the thickness of the primary particles is below 0.05 µm, flat particles suitable for a cosmetic of 5 µm to 10 µm that can exhibit lubricity are not formed. If the thickness of the primary particles exceeds 0.5 µm, the transparency is lower and the plane surface cannot be maintained smoothly in the case where the cosmetic is applied to spread on the skin.

In the present invention, the proportion of the boron nitride powder in the cosmetic pigment is preferably 0.1 mass% to 70 mass%. If the proportion is below 0.1 mass%, the effect of improving the sustainability and the adhesion as desired by the present invention is poor. If the proportion exceeds 70 mass%, the glittering appearance specific to the BN powder intensifies, and appropriate gloss cannot be attained.

The manufacturing method according to the present invention is described next.

In the present invention, a high-purity BN powder having a turbostratic structure is prepared as a raw material. The BN powder having a turbostratic structure in the present invention means the BN powder that has an incompletely-crystallized structure which exhibits a X-ray diffraction pattern not with a sharp peak corresponding to a hexagonal system but with a broad peak..

Such a BN powder can be obtained by uniformly mixing boric acid and/or its dehydration product, urea and/or its compound (dicyandiamide, melamine, etc.), and boron carbide (B₄C) and heating the mixture in an inert gas atmosphere.

The obtained BN powder is then heat-treated at a temperature of 1500 °C to 2300 °C in an inert gas atmosphere and, after grinding, boric acid is removed by washing. The result is then heat-treated at a temperature of 300 °C or higher in a non-oxidizing, reduced-pressure atmosphere with a furnace pressure less than or equal to 0.01 MPa, to effectively reduce functional groups on the powder surface and achieve high water repellency and high oil absorbency.

Here, since BN easily combines with oxygen, the atmosphere in the heating treatment before grinding is set to an inert gas atmosphere to prevent such combination.

Moreover, the heating temperature of 1500 °C to 2300 °C is set for the following reason. If the treatment temperature is below 1500 °C, a powder with sufficiently grown crystals cannot be obtained. If the treatment temperature exceeds 2300 °C, defects are likely to occur and result in lower transparency.

The heating temperature in the heating treatment for removing functional groups is set to 300 °C or higher, because a temperature of at least 300 °C is needed to completely remove the organic dispersant. The upper limit of the heating temperature is not particularly limited, and may be adequately set to about 2300 °C.

The atmosphere in this treatment is set to a non-oxidizing, reduced-pressure atmosphere less than or equal to 0.01 MPa, in order to effectively eliminate the separated functional groups from the system and prevent reoxidation and adsorption.

A BN powder that is flat-shaped and slidable, has high water repellency and high oil absorption, and is ideal as an extender for powder foundation can be obtained in this way.

The BN powder according to the present invention is effective mainly for use as a cosmetic pigment for powder foundation, but the following other uses are also possible.

The BN powder is suitable for use in makeup cosmetics such as face powder, base, face color, cheek rouge, and eye shadow, and skin care cosmetics such as sunscreen, milky lotion, and beauty essence.

The basic components of the above-mentioned cosmetics are not particularly limited, and may be conventionally well-known components so long as the BN powder according to the present invention is used instead of a BN powder or an inorganic powder (e.g. silicic anhydride, aluminum oxide, titanium oxide, zinc oxide, zirconium oxide) in the conventional components.

### EXAMPLES

The following describes examples of the present invention.

### (Example 1)

100 parts by mass boric acid, 100 parts by mass melamine, and 10 parts by mass boron carbide were uniformly mixed in a mixing machine, and heated in an inert atmosphere to obtain a boron nitride powder having a turbostratic structure. The obtained boron nitride powder was then heated to 2000 °C in a nitrogen atmosphere, to obtain a BN powder bulk body.

The obtained product was identified by an X-ray diffractometer and as a result determined to be highly crystalline BN.

After this, the BN powder bulk body was grinded in a pin mill device, and washed and dried to reduce the amount of B to less than or equal to 100 ppm (conventional example).

Following this, the obtained powder was heat-treated at 1000 °C for 10 hours in a nitrogen atmosphere reduced in pressure to 0.005 MPa (No. 1).

The obtained powder was also heat-treated at 600 °C for 10 hours in a nitrogen atmosphere reduced in pressure to 0.005 MPa (No. 2).

Table 1 shows the quality evaluation results of each of the obtained BN powders.

Table 1 also shows the results of examining the water repellency, the oil absorption, the amount of soluble B, and the pH of each of the obtained BN powders.

The results of the same examination on the conventional BN powder and talc are also shown in Table 1 for comparison.

The water repellency, the oil absorption, and the amount of soluble B of the BN powder were each measured as follows.

### (1) Water repellency

A water permeability test conforming to JIS A 6909 (Water Permeability Test B Method) was conducted to measure the water permeation speed.

In detail, using a powder wetting permeation analyzer PW-500 (made by Mitsuwa Frontech Corp.), 1 g powder was charged into a column of 10 mm in inside diameter, and the "wetting height" from the lower liquid contact surface was measured with time, to calculate the permeation speed.

### (2) Oil absorption

The oil absorption was measured by a test conforming to "Oil absorption" defined in JIS K 5101.

In detail, 2 g powder was metered on a watch glass, and refined linseed oil was added drop by drop from a burette. Upon each addition, the added linseed oil was kneaded using a spatula. This is repeated until the hardness reaches a smoothness level with no cracking or separation. The value obtained by converting the measurement to correspond to 100 g powder was set as the oil absorption.

### (3) Amount of soluble B

The amount of soluble B was measured in conformance with the Japanese Standards of Quasi-drug Ingredients 2006.

In detail, 2.5 g powder was metered in a Teflon® beaker, to which 10 ml ethanol was added and mixed well. After newly adding 40 ml boiled and cooled water and mixing them, the mixture was heated at 50 °C for 1 hour. The liquid was then filtered and B in the filtrate was measured.

### (4) pH

The pH was measured in conformance with the Japanese Standards of Quasi-drug Ingredients 2006.

In detail, 10 ml ethanol was added to 2.5 g powder and mixed well. After newly adding 50 ml boiled and cooled water and mixing them, the mixture was filtered and the pH of the filtrate was measured.

[Table 1]

**Table 1**

| | Extender | | | |
|---|---|---|---|---|
| | No. 1 | No. 2 | Conventional BN | Talc * |
| BN (%) | 99.9 | 99 | 99 | - |
| Average particle diameter (µm) | 5.8 | 8.6 | 5.4 | 5.2 |
| Oxygen content (mass%) | 0.1 | 0.5 | 0.8 | - |
| Specific surface area (m²/g) | 3.5 | 2.5 | 4.5 | 8.5 |
| Permeation speed (mm²/s) | 0 | 0.8 | 0.5 | 1.2 |
| Oil absorption (ml/100g) | 353 | 107 | 86 | 72 |
| Amount of soluble (ppm) | 12 | 8 | 78 | - |
| pH | 6.9 | 6.7 | 6.8 | 9.1 |

| | | | | |
|---|---|---|---|---|
| * Nippon Talc P-3 | | | | |

As shown in Table 1, each of the BN powders (Nos. 1 and 2) obtained according to the present invention has high water repellency and oil absorption and a low amount of soluble B, and is excellent in sustainability and spreadability.

### (Example 2)

Various cosmetics of Invention Examples 1 to 6 and Comparative Examples 7 to 13 shown below were produced using the boron nitride powders shown in Table 1.

### Invention Example 1 (powder foundation)

| (composition) | (mix proportion %) |
|---|---|
| - boron nitride (No.1 in Table 1) | 20.0 |
| - N-lauroyl lysine-treated (5%) red iron oxide | 1.0 |
| - N-lauroyl lysine-treated (5%) yellow iron oxide | 4.0 |
| - N-lauroyl lysine-treated (5%) black iron oxide | 0.5 |
| - perfluoroalkyl phosphoric acid diethanolamine-treated titanium oxide (#1) | 10.0 |
| - silicone (2%)-treated fine particulate titanium oxide | 2.0 |
| - N-lauroyl lysine-treated (5%) sericite | 29.0 |
| - perfluoroalkyl phosphoric acid diethanolamine-treated synthetic phlogopite | 10.0 |
| - perfluoroalkyl phosphoric acid diethanolamine-treated talc | 10.0 |
| - cross-linked type silicone powder (Trefil E-505C made by Dow Corning Toray Co., Ltd.) | 0.3 |
| - urethane powder (PLASTIC POWDER CS-400 made by Toshiki Pigment Co., Ltd.) | 2.0 |
| - methylparaben | 0.1 |
| - sodium dehydroacetate | 0.1 |
| - methylpolysiloxane (KF-96A (6CS) made by Shin-Etsu Chemical Co., Ltd.) | 4.0 |
| - diisostearyl malate | 1.5 |
| - glyceryl tri-2-ethylhexanate | 2.0 |
| - vaseline | 0.5 |
| - 2-ethylhexyl paramethoxycinnamate | 3.0 |

| | |
|---|---|
| (#1) TIPAQUE CR-50 (made by Ishihara Sangyo Kaisha, Ltd.) coated with perfluoroalkyl phosphoric acid diethanolamine (5%). | |

### Invention Example 2 (solid face powder)

| (composition) | (mix proportion %) |
|---|---|
| - boron nitride (No. 2 in Table 1) | 15.0 |
| - silicone-treated (2%) red iron oxide | 0.3 |
| - silicone-treated (2%) yellow iron oxide | 0.5 |
| - silicone-treated (2%) black iron oxide | 0.05 |
| - silicone-treated titanium oxide (#2) | 5.0 |
| - silicone-treated zinc oxide | 1.0 |
| - (iron oxide/titanium oxide) sintered material | 1.0 |
| - polyalkyl acrylate (GBX-10S made by Ganz Chemical Co., Ltd.) | 3.0 |
| - silk powder | 1.0 |
| - platy barium sulfate | 35.0 |
| - silicone-treated (2%) talc | 31.75 |
| - methylparaben | 0.1 |
| - sodium dehydroacetate | 0.1 |
| - vaseline | 1.0 |
| - dimethylpolysiloxane | 1.0 |
| - glyceryl tri-2-ethylhexanate | 2.0 |
| - isononyl isononanoate | 2.0 |
| - octyldodecanol | 1.0 |

| | |
|---|---|
| (#2) 2% silicon-treated TIPAQUE CR-50 (made by Ishihara Sangyo Kaisha, Ltd.). | |

### Invention Example 3 (powdery foundation)

| (composition) | (mix proportion %) |
|---|---|
| - boron nitride (No. 1 in Table 1) | 20.0 |
| - silicon-treated (2%) red iron oxide | 0.4 |
| - silicon-treated (2%) yellow iron oxide | 1.0 |
| - silicon-treated (2%) black iron oxide | 0.2 |
| - silicon-treated titanium oxide | 8.0 |
| - N- lauroyl lysine powder | 15.0 |
| - mica titanium | 4.0 |
| - talc | 27.2 |
| - Cellulose Cellulobeads D-5 (made by Daito Kasei Kogyo Co., Ltd.) | 5.0 |
| - cornstarch (Nisshoku Cornstarch | |
| made by Nihon Shokuhin Kako Co., Ltd.) | 15.0 |
| - methylparaben | 0.1 |
| - sodium dehydroacetate | 0.1 |
| - liquid paraffin | 1.5 |
| - methylphenylpolysiloxane (FZ-209 made by Dow Corning Toray Co., Ltd.) | 2.0 |
| - vaseline | 0.5 |

### Invention Example 4 (powder eye shadow)

| (composition) | (mix proportion %) |
|---|---|
| - boron nitride (No. 2 in Table 1) | 25.0 |
| - isooctyl isononanoate | 5.0 |
| - hexyl oxystearate | 8.0 |
| - glyceryl trioctanoate | 4.0 |
| - vaseline | 1.0 |
| - Red No. 226 | 1.0 |
| - ultramarine | 5.0 |
| - mica titanium | 10.0 |
| - iron blue-treated mica titanium | 8.0 |
| - titanium oxide-coated glass flake | 2.0 |
| - titanium oxide-coated synthetic phlogopite | 1.0 |
| - nylon powder | 5.0 |
| -talc | 15.0 |
| - silicon-treated (2%) sericite | 10.0 |

### Invention Example 5 (solid foundation)

| (composition) | (mix proportion %) |
|---|---|
| - boron nitride (No. 1 in Table 1) | 5.0 |
| - organic titanate-treated red iron oxide | 0.2 |
| - organic titanate-treated yellow iron oxide | 0.5 |
| - organic titanate-treated black iron oxide | 0.05 |
| - silicic anhydride | |
| (Sunsphere H-122 made by Asahi Glass Co., Ltd.) | 5.0 |
| - organic titanate-treated titanium oxide (#3) | 3.0 |
| - silicone-treated fine particulate zinc oxide | 2.0 |
| - low melting point paraffin | 10.0 |
| - silicone gel | |
| (KSG-16 made by Shin-Etsu Chemical Co., Ltd.) | 2.0 |
| - 2-ethylhexyl paramethoxycinnamate | 1.0 |
| - methylparaben | 0.2 |
| - phenoxyethanol | 0.1 |
| - isocetyl myristate | remaining amount |

| | |
|---|---|
| (#3) TIPAQUE CR-50 (made by Ishihara Sangyo Kaisha, Ltd.) coated with organic titanate. | |

### Comparative Examples 1 to 5

Respective cosmetics having the compositions of the above-mentioned Invention Examples 1 to 5 except boron nitride.

### Comparative Example 6

A cosmetic in which the conventional boron nitride shown in Table 1 is used instead of the boron nitride of Invention Example 1.

Table 2 shows the results of examining the sustainability and the spreadability of each of these cosmetics.

To evaluate the above-mentioned various properties, a research panel of 20 cosmetic evaluation specialists used the present invention products and the comparative products and made evaluation on the following 5-point scale. The average score of the whole research panel was then calculated, and each product was rated according to the following four levels.

### Evaluation scale

5: very good
4: good
3: fair
2: poor
1: very poor
Rating scale
level A: greater than or equal to 4.5
level B: greater than or equal to 3.5, and less than 4.5
level C: greater than or equal to 2.5, and less than 3.5
level D: less than 2.5

[Table 2]

**Table 2**

| | Sustainability | Spreadability |
|---|---|---|
| Invention Example 1 | A | A |
| Invention Example 2 | A | A |
| Invention Example 3 | A | A |
| Invention Example 4 | A | A |
| Invention Example 5 | A | A |
| Comparative Example 1 | B | C |
| Comparative Example 2 | B | C |
| Comparative Example 3 | B | C |
| Comparative Example 4 | B | C |
| Comparative Example 5 | B | C |
| Comparative Example 6 | C | C |

As shown in Table 2, the products using any of the BN powders according to the present invention as a cosmetic extender are rated higher than the conventional products, in both sustainability and spreadability.

## Claims

1. A highly water repellent and highly oil absorbent boron nitride powder comprising flat-shaped primary particles of BN and an aggregate of the primary particles,
wherein a water permeation speed is less than 1 mm²/s, and oil absorption is 100 ml/100 g to 500 ml/100 g.

2. The highly water repellent and highly oil absorbent boron nitride powder according to claim 1,
wherein the primary particles of BN are flat-shaped with an average major diameter of 2 µm to 20 µm and a thickness of 0.05 µm to 0.5 µm.

3. The highly water repellent and highly oil absorbent boron nitride powder according to claim 1 or 2,
wherein an amount of soluble boron is less than or equal to 100 ppm.

4. The highly water repellent and highly oil absorbent boron nitride powder according to any of claims 1 to 3,
wherein a specific surface area is 1 m²/g to 10 m²/g, and an oxygen content is less than or equal to 1.5 mass%.

5. A method for manufacturing a highly water repellent and highly oil absorbent boron nitride powder, the method comprising:
heating at least one of boric acid and a dehydration product thereof, at least one of urea and a compound thereof, and boron carbide in an inert atmosphere to obtain a boron nitride powder having a turbostratic structure;
heat-treating the obtained boron nitride powder at a temperature of 1500 °C to 2300 °C in an inert atmosphere;
grinding the heat-treated boron nitride powder;
washing the ground boron nitride powder to remove boric acid; and
heat-treating the washed boron nitride powder at a temperature of 300 °C or higher in a non-oxidizing, reduced-pressure atmosphere with a furnace pressure less than or equal to 0.01 MPa.

6. A cosmetic comprising the boron nitride powder according to any of claims 1 to 4.

7. The cosmetic according to claim 6,
wherein an amount of the boron nitride powder contained in the cosmetic is 0.1 mass% to 70 mass%.

8. The cosmetic according to claim 6 or 7,
wherein the cosmetic is powder foundation.
